# FASCICULE DE BREVET EUROPEEN

(11) **EP 3 577 711 B1**
(45) Date de publication et mention de la délivrance du brevet: **03.03.2021**
(21) Numéro de dépôt: 18715218.6
(22) Date de dépôt: 21.03.2018
(51) Int. Cl.: H01M 10/0569, H01M 10/052, H01M 4/587, H01M 10/0568

(54) **NOUVEAUX ÉLECTROLYTES À BASE DE LIQUIDES IONIQUES UTILISABLES POUR DES DISPOSITIFS DE STOCKAGE ÉLECTROCHIMIQUE**
NEUARTIGE ELEKTROLYTEN AUF BASIS VON IONISCHEN FLÜSSIGKEITEN, DIE IN ELEKTROCHEMISCHEN SPEICHERVORRICHTUNGEN ANWENDBAR SIND
NOVEL ELECTROLYTES BASED ON IONIC LIQUIDS USABLE IN ELECTROCHEMICAL STORAGE DEVICES

(30) Priorité: 23.03.2017 FR 1752435
(43) Date de publication de la demande: 11.12.2019
(73) Titulaire: Commissariat à l'Énergie Atomique et aux Énergies Alternatives, 75015 Paris (FR)
(72) Inventeur: CADRA, Stéphane, 37550 Saint Avertin (FR); SZYMCZAK, Jonathan, 29000 Quimper (FR); LE DIGABEL, Matthieu, 37260 Monts (FR); BILLER, Agnès, 37550 Saint-Avertin (FR)
(74) Mandataire: Brevalex
(86) Numéro de dépôt international: PCT/FR2018/050683
(87) Numéro de publication internationale: WO 2018/172697

(56) Documents cités:
- EP-A1- 2 549 577
- WO-A1-2010/023185
- FR-A1- 3 002 086
- US-A1- 2015 340 738
- S. SEKI ET AL: "Compatibility of N-Methyl-N-propylpyrrolidinium Cation Room-Temperature Ionic Liquid Electrolytes and Graphite Electrodes", JOURNAL OF PHYSICAL CHEMISTRY C, vol. 112, no. 42, 1 octobre 2008 (2008-10-01), pages 16708-16713, XP007906837, ISSN: 1932-7447, DOI: 10.1021/JP805403E [extrait le 2008-10-01]

## Description

### DOMAINE TECHNIQUE

La présente invention a trait à de nouveaux électrolytes à base d'au moins deux liquides ioniques et d'au moins un sel de lithium, ces électrolytes bénéficiant notamment d'une température de fusion sub-ambiante et d'une conductivité ionique élevée (par exemple, supérieure à 1 mS/cm). En outre, au moins un des liquides ioniques spécifiques entrant dans la composition des électrolytes de l'invention présente une fonction stabilisante vis-à-vis de certains matériaux classiquement employés pour la constitution d'électrodes, telles que des électrodes en graphite. De tels électrolytes présentent également un intérêt pour une utilisation dans des batteries secondaires, notamment en raison des faibles pertes de capacité lors du fonctionnement desdites batteries et d'un bon rendement de restitution de l'énergie stockée lors de la charge (soit, en d'autres termes, une bonne efficacité coulombique).

Aussi de par toutes ses propriétés électrochimiques, les électrolytes de l'invention peuvent trouver application dans tous les domaines en lien avec le stockage électrochimique de l'énergie, tels que les accumulateurs (ou batteries secondaires) ou, plus spécifiquement, les accumulateurs mettant en jeu au moins une électrode à base de graphite.

Les liquides ioniques sont des sels spécifiques se présentant à l'état liquide à température ambiante (le point de fusion étant inférieur aux températures ambiantes, par exemple 20°C), par opposition à des sels classiques, comme le chlorure de sodium, qui présentent un point de fusion proche de 180°C, ces liquides ioniques pouvant être représentés par la formule générale suivante :

A⁺X⁻

dans laquelle :
*A⁺ représente un cation, tel qu'un cation phosphonium ou un cation ammonium quaternaire ; et
*X⁻ représente un anion organique, tel qu'un anion imidure.

La particularité des liquides ioniques en termes d'état provient, notamment, de la différence morphologique entre l'anion et le cation (par exemple, au niveau de l'encombrement stérique et de la géométrie) peu favorable à l'établissement d'une forme cristalline du sel.

En outre, les liquides ioniques présentent une faible toxicité, une très faible inflammabilité, une stabilité électrochimique et une conductivité ionique intéressante.

De ce fait, les liquides ioniques présentent un grand intérêt dans les domaines nécessitant la mise en œuvre de solutions conductrices d'ions et peuvent être notamment utilisés comme électrolytes de dispositifs à stockage d'énergie, tels que les batteries sécuritaires de dernière génération, comme les batteries lithium-soufre, les batteries lithium-ion ou encore les batteries à flux redox.

Parmi l'ensemble des liquides ioniques existants, les plus répandus et utilisés pour une utilisation en batterie sont de type *bis*(trifluorométhanesulfonyl)imidure de N,N'-méthyl-alkyl pyrrolidinium (le groupe alkyle pouvant être un groupe n-propyle ou un groupe n-butyle), en raison de leurs bonnes performances de cyclage en système Li-ion, comme l'explique Kim et al. dans J. Power Sources, 199 (2012) 239-246*.*

Toutefois, dans le domaine des batteries, le point limitant de ces liquides ioniques reste leur haute viscosité et leur incompatibilité vis-à-vis de certains matériaux d'électrodes, comme cela est le cas avec le graphite, ce qui induit une limitation des performances des batteries en terme de cyclage, le cyclage désignant communément le nombre de cycles de charge/décharge qui peut être effectué par une batterie. Ce phénomène est notamment lié à l'exfoliation électrochimique entrant en jeu lorsqu'une électrode de graphite est mise en contact avec un liquide ionique. Un tel phénomène a notamment été décrit par Lu et al., dans ACS nano, 3, 8, 2367-2375*,* afin d'obtenir facilement des nanoparticules de carbone et graphène à partir de graphite.

Afin de concevoir un électrolyte à base de liquides ioniques disposant de bonnes performances de cyclage, certains auteurs ont eu recours à des additifs d'électrolyte, dont la réactivité particulière permet de stabiliser le fonctionnement d'une cellule électrochimique. La plupart des additifs d'électrolyte ont la capacité de former un dépôt protecteur (SEI) à la surface des électrodes, tel que cela est le cas du carbonate de vinylidène employé dans ce but par Holzapfel et al. (Carbon, 47 (2005) 1488-1498) dans ses formulations d'électrolytes à base de liquides ioniques.

Par ailleurs, US 2015/340738 décrit une méthode de modification de la solubilité des anions polysulfures dans des batteries soufre-lithium par le biais d'un électrolyte comprenant des liquides ioniques fonctionnalisés, lesquels liquides ioniques fonctionnalisés répondent à la formule C⁺A⁻, dans laquelle :
-A⁻ est un anion choisi dans le groupe constitué par les halogénures, les nitrates, les phosphates, les imidures, les borates, les phosphazines, les acétates et les sulfonates ;
-C⁺ est un cation choisi parmi les ammoniums, les sulfoniums, les phosphoniums ; les noyaux hétérocycliques à 5 ou 6 chaînons comprenant de 1 à 3 hétéroatomes (azote, oxygène et soufre), dans lequel un ou plusieurs des atomes portés par le noyau est (sont) substitué(s) par un ou plusieurs groupes ou atomes, tels que des halogènes, de l'oxygène, de l'azote, du soufre, du phosphore, des alcanes, des esters, des éthers, des cétones, des carbonyles, des alcoxyalcanes, des alcènes, des alcynes, des aryles, des nitriles, des silanes, des sulfones, des thiols, des phénols, des hydroxyles, des amines, des imines, des aldéhydes, des acides carboxyliques, des carbonates et des anhydrides d'acides, chacun de ces groupes pouvant être également substitué ;
et comprenant un ou plusieurs sels alcalins et un ou plusieurs co-solvants organiques.

FR 3 002 086 décrit un procédé de synthèse de liquides ioniques à groupe fonctionnel carbonate et aux liquides ioniques obtenus par ce procédé, les liquides ioniques obtenus comprenant, comme cations, des cations comprenant un groupe chargé positivement imidazolium, pyrrolidinium ou ammonium, lequel groupe étant lié à un groupe carbonate, un exemple spécifique illustré étant le composé de formule suivante :

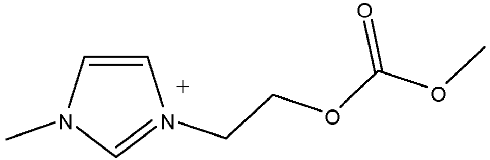

EP 2 549 577 a trait à des batteries secondaires au lithium, qui utilisent une solution électrolytique à propriétés retardatrices de flamme, ladite solution comprenant un liquide ionique contenant des anions *bis*(fluorosulfonyl)imidures.

Des exemples concrets de cations susceptibles d'entrer dans la constitution de ces liquides ioniques sont des cations alkylammoniums, tels que le triméthyl-N-propylammonium, le triéthylammonium ; des cations imidazoliums, tels que l'éthylméthylimidazolium, le butyméthylimidazolium ; des cations pyrrolidiniums, tels que le N-méthyl-N-propylpyrrolidinium et des cations pipéridiniums, tels que le N-méthyl-N-propylpipéridinium.

WO 2010/023185 a trait à des accumulateurs lithium-ion comprenant :
- une électrode négative comprenant, comme matériau actif, du graphite ;
- une électrode positive comprenant, comme matériau actif, du LiFePO₄ ;
- un électrolyte liquide comprenant au moins un liquide ionique de formule C⁺A⁻ avec C⁺ représentant un cation et A⁻ représentant un anion, et au moins un sel conducteur, l'électrolyte liquide comprenant, en outre, un additif organique carbonate de vinyléthylène.

Plus spécifiquement, le cation C⁺ du liquide ionique décrit dans ce document est choisi parmi les imidazoliums non substitués ou substitués, tels que les tri-, tétra- et pentaalkyl imidazoliums, les ammoniums quaternaires, les pipéridiniums non substitués ou substitués tels que les dialkylpipéridiniums, les pyrrolidiniums non substitués ou substitués tels que les dialkylpyrrolidiniums, les pyrazoliums non substitués ou substitués tels les dialkylpyrazoliums, les pyridiniums non substitués ou substitués, tels que les alkylpyridiniums, les phosphoniums tels que les tétraalkylphosphoniums, les sulfoniums, tels que les trialylsulfoniums.

J. Phys.Chem., C 2008, 112, 16708-16713 décrit l'utilisation de liquides ioniques dans des accumulateurs comprenant une électrode à base de graphite, ces liquides ioniques comprenant un cation N-méthyl-N-propylpyrrolidinium.

Aussi, au vu de ce qui existe, les auteurs de la présente invention se sont fixé pour but de mettre au point de nouveaux électrolytes qui, outre le fait de présenter une bonne conductivité ionique, permettent une stabilisation de l'interface électrode-électrolyte, notamment lorsque ces électrolytes sont utilisés dans un dispositif de stockage électrochimique comprenant une électrode comprenant du graphite. Ces nouveaux électrolytes, une fois incorporés dans ces dispositifs, doivent contribuer également à minimiser la perte de capacité en cours de cyclage et un bon rendement de restitution de l'énergie stockée lors de la charge (soit, en d'autres termes, une bonne efficacité coulombique).

### EXPOSÉ DE L'INVENTION

L'invention a ainsi trait à un électrolyte comprenant au moins un sel de lithium et au moins deux liquides ioniques, dont au moins l'un est un liquide ionique résultant de l'association d'au moins un cation répondant à la formule (I) suivante : 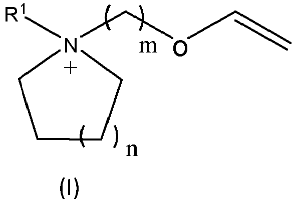 dans laquelle :
- R¹ est un groupe hydrocarboné acyclique ;
- n est un entier allant de 0 à 3 ;
- m est un entier allant de 1 à 4 ;
et d'au moins anion Y.

Il s'entend, de la formulation ci-dessus, que le sel de lithium susmentionné n'est pas un liquide ionique et que les deux liquides ioniques sont des liquides ioniques distincts, dont l'un au moins est un liquide ionique résulte de l'association d'un cation de formule (I) et d'au moins un anion Y.

Il s'entend que le ou les cations de formule (I) et le ou les anions Y sont associés de sorte à assurer l'électroneutralité du liquide ionique résultant (en d'autres termes, un liquide ionique dont la ou les charges positives du ou desdits cations équilibrent la ou les charges négatives du ou desdits anions).

De manière plus explicite, le cation de formule (I) peut correspondre, selon les valeurs de n, à l'une des formules suivantes :
*pour n=0, la formule (Ia) suivante : 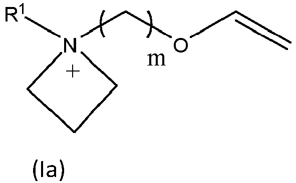
*pour n=1, la formule (Ib) suivante : 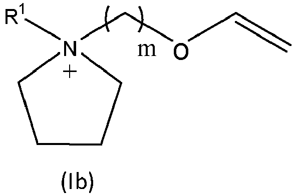
*pour n=2, la formule (Ic) suivante : 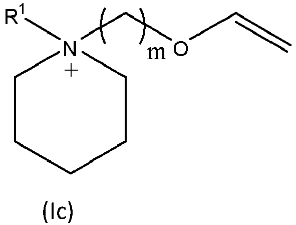
*pour n=3, la formule (Id) suivante :

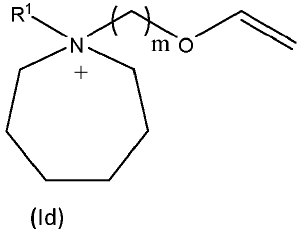

De manière avantageuse, l'un des liquides ioniques des électrolytes de l'invention est un liquide ionique, dans lequel le cation est un cation de formule (I) avec n étant égal à 1 (soit, en d'autres termes, un cation de formule (Ib)).

Le groupe R¹ est un groupe hydrocarboné acyclique et, plus spécifiquement il peut s'agir d'un groupe hydrocarboné acyclique, linéaire ou ramifié, tel qu'un groupe alkyle, comportant de 1 à 4 atomes de carbone. Encore plus spécifiquement, le groupe R¹ peut être un groupe de formule -CₚH₂ₚ₊₁, avec p étant un entier allant de 1 à 4, par exemple, un groupe méthyle, un groupe éthyle, un groupe n-propyle, un groupe isopropyle, un groupe n-butyle, un groupe isobutyle, un groupe tertiobutyle.

A titre d'exemple, R¹ peut être un groupe méthyle.

A titre d'exemple, m peut être égal à 2.

L'anion Y peut être un anion (en d'autres termes, le contre-ion associé au cation de formule (I)) choisi parmi les anions halogénures (par exemple, chlorure, bromure ou iodure), un anion nitrate, un anion phosphate, les anions imidures et plus spécifiquement, un anion nitrate, un anion phosphate ou un anion imidure.

Plus spécifiquement :
- lorsque l'anion est un anion nitrate, celui-ci répond à la formule NO₃⁻ ;
- lorsque l'anion est un anion phosphate, celui-ci répond à la formule PO₄³⁻ ;
- lorsque l'anion est un anion imidure, cela signifie, classiquement, qu'il comporte un radical imide, dont la charge négative est portée par l'atome d'azote, lequel atome d'azote est lié à deux groupes carbonyles ou deux groupes sulfonyles, ledit radical imide pouvant être représenté par l'une des formules (II) et (III) suivantes : 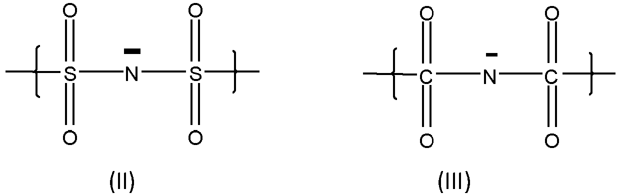
les accolades indiquant que les groupes -SO₂- et -CO₂- sont liés à d'autres groupes.

Avantageusement, l'anion est un anion imidure, dont la charge négative est portée par l'atome d'azote, lequel atome d'azote est lié à deux groupes sulfonyles, un tel anion pouvant être représenté par la formule générale (II') suivante : 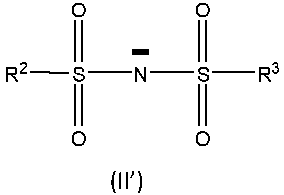 dans laquelle R² et R³ représentent, indépendamment l'un de l'autre, un atome de fluor ou un groupe perfluorocarboné.

Plus spécifiquement, R² et R³ peuvent représenter, tous deux, un atome de fluor ou, tous deux, un groupe perfluorocarboné, par exemple, un groupe perfluorométhyle -CF₃.

Des anions imidures particuliers répondant à ces spécificités sont ceux de formules (IV) et (V) suivantes : 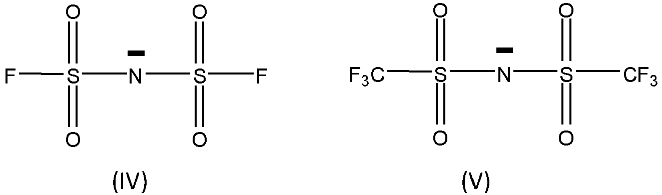 ces anions imidures pouvant être qualifiés respectivement de *bis*(fluorosulfonyl)imidure et de *bis*(trifluorométhanesulfonyl)imidure.

Plus spécifiquement encore, l'un des liquides ioniques des électrolytes de l'invention est un liquide ionique, dans lequel le cation est un cation de formule (I) avec n étant égal à 1 (soit, en d'autres termes, un cation de formule (Ib)) avec R¹ correspondant à un groupe méthyle et m étant égal à 2 et l'anion imidure étant un anion *bis*(fluorosulfonyl)imidure ou *bis*(trifluorométhanesulfonyl)imidure.

Dans ce cas, le cation répond à la formule (IIb) suivante :

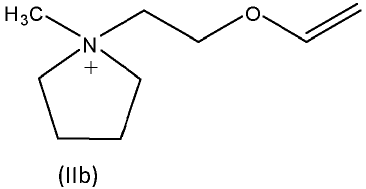

Ces liquides ioniques résultent ainsi de l'association d'un cation de formule (IIb) et d'un anion de formule (IV) ou (V), lesquels liquides ioniques répondent aux formules respectives (VI) et (VII) suivantes : 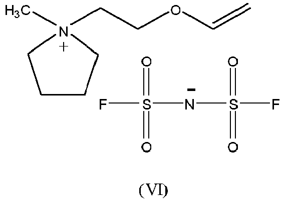 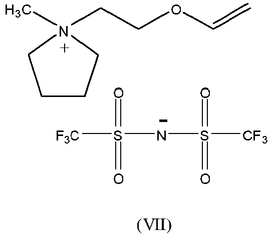 ces liquides ioniques pouvant être nommés respectivement *bis*(fluorosulfonyl)imidure de N-(méthyl)-(2-vinyloxyéthyl)pyrrolidinium et *bis*(trifluorométhanesulfonyl)imidure de N-(méthyl)-(2-vinyloxyéthyl)pyrrolidinium.

Comme mentionné ci-dessus, l'un au moins des liquides ioniques est un liquide ionique résultant de l'association d'au moins un cation répondant à la formule (I) définie ci-dessus et d'un anion Y, ce ou ces liquides ioniques représentant, avantageusement, 5 à 20% du volume total des liquides ioniques présents dans l'électrolyte, ce qui signifie, dans ce cas, que l'électrolyte comprend au moins un autre liquide ionique dont la définition ne répond pas à celle pour lequel le cation répond à la formule (I) ci-dessus et l'anion est un anion Y.

Aussi, dans les électrolytes de l'invention, l'un au moins des liquides ioniques peut être un liquide ionique différent de ceux pour lesquels le cation est un cation de formule (I) telle que définie ci-dessus et l'anion est un anion Y.

De manière plus spécifique, dans les électrolytes de l'invention, l'un au moins des liquides ioniques est un liquide ionique résultant de l'association d'un cation phosphonium, sulfonium, azétidinium, pyrrolidinium ou pipéridinium et d'un anion halogénure, phosphate, nitrate ou imidure, étant entendu que, lorsque le cation est un cation azétidinium, pyrrolidinium ou pipéridinium, le cation ne répond pas à la formule (I) définie ci-dessus.

De préférence, l'anion est un anion imidure.

De préférence, le cation est un cation pyrrolidinium ou pipéridinium.

Lorsque le cation est un cation azétidinium, pyrrolidinium ou pipéridinium, il s'entend que le cation ne répond pas à la formule (I) susmentionnée. En outre, avantageusement, ce cation ne comprend pas de groupe éthylénique.

Un tel cation peut répondre à la formule (VIII) suivante : 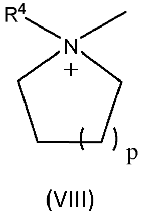 dans laquelle :
- R⁴ est un groupe hydrocarboné acyclique ; et
- p est un entier allant de 0 à 2.

De manière plus explicite, le cation de formule (VIII) peut correspondre, selon les valeurs de p, à l'une des formules suivantes :
*pour p=0, un cation azétidinium spécifique de formule (VIIIa) suivante : 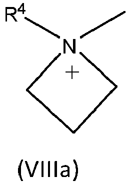
*pour p=1, un cation pyrrolidinium spécifique de formule (VIIIb) suivante : 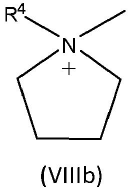
*pour p=2, un cation pipéridinium spécifique de formule (VIIIc) suivante :

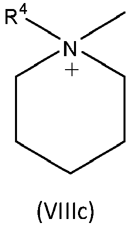

Quant aux anions, ils peuvent répondre aux mêmes définitions que celles mentionnées plus haut au sujet des liquides ioniques comportant un cation de formule (I).

De manière avantageuse, l'un des liquides ioniques des électrolytes de l'invention est un liquide ionique, dans lequel le cation est un cation de formule (VIII) avec p étant égal à 1 (soit, en d'autres termes, un cation de formule (Vlllb)).

Le groupe R⁴ est un groupe hydrocarboné acyclique et, plus spécifiquement il peut s'agir d'un groupe hydrocarboné acyclique, linéaire ou ramifié, tel qu'un groupe alkyle, comportant de 1 à 4 atomes de carbone. Encore plus spécifiquement, le groupe R⁴ peut être un groupe de formule -CₚH₂ₚ₊₁, avec p étant un entier allant de 1 à 4, par exemple, un groupe méthyle, un groupe éthyle, un groupe n-propyle, un groupe isopropyle, un groupe n-butyle, un groupe isobutyle, un groupe tertiobutyle.

A titre d'exemple, R⁴ peut être un groupe n-propyle.

Plus spécifiquement encore, l'un des liquides ioniques des électrolytes de l'invention est un liquide ionique, dans lequel le cation est un cation de formule (VIII) avec p étant égal à 1 (soit, en d'autres termes, un cation de formule (Vlllb)) avec R⁴ correspondant à un groupe n-propyle et l'anion imidure est un anion *bis*(trifluorométhanesulfonyl)imidure.

Dans ce cas, le cation répond à la formule (IX) suivante :

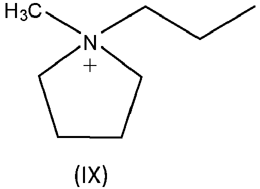

Ce liquide ionique résulte ainsi de l'association d'un cation de formule (IX) et d'un anion de formule (V), lequel liquide ionique répondant à la formule (X) suivante : 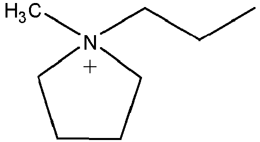 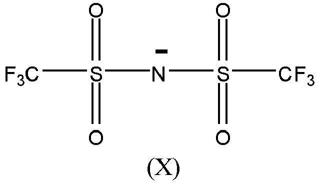 ce liquide ionique pouvant être nommé *bis*(trifluorométhanesulfonyl)imidure de N,N-(méthyl)-(propyl)-pyrrolidinium.

En outre, les électrolytes de l'invention comprennent au moins un sel de lithium.

A titre d'exemples de sel de lithium, on peut citer l'hexafluorophosphate de lithium (LiPF₆), le tétrafluoroborate de lithium (LiBF₄), le *bis*(trifluorométhanesulfonyl)imidure de lithium (connu sous l'abréviation LiTFSI), l'hexafluoroarsénate de lithium (LiAsF₆), le nitrate de lithium (LiNO₃) ou encore le perchlorate de lithium (LiClO₄).

Le sel de lithium peut être compris dans les électrolytes de l'invention à hauteur de 0,1 à 2 moles de sel par litre de liquides ioniques.

Selon un mode de réalisation de l'invention, des électrolytes particuliers de l'invention peuvent comprendre voire être constitués exclusivement :
- d'un liquide ionique résultant de l'association d'au moins un cation répondant à la formule (I) telle que définie ci-dessus et d'au moins un anion Y (par exemple, un liquide ionique résultant de l'association d'un cation de formule (Ib) telle que définie ci-dessus et d'un anion imidure) ;
- d'un liquide ionique différent de ceux pour lesquels le cation est un cation de formule (I) telle que définie ci-dessus et l'anion est un anion Y, (par exemple, un liquide ionique résultant de l'association d'un cation de formule (VIIIb) telle que définie ci-dessus et d'un anion imidure) ; et
- d'un sel de lithium.

Plus spécifiquement, un électrolyte particulier conforme à l'invention est un électrolyte comprenant :
- un liquide ionique de formule (VI) telle que définie ci-dessus ;
- un liquide ionique de formule (X) telle que définie ci-dessus ;
- un sel de lithium *bis*(trifluorométhanesulfonyl)imidure de lithium.

Les électrolytes de l'invention présentent tout particulièrement un intérêt pour les systèmes de stockage électrochimique, dont l'une des électrodes comporte du graphite. En effet, il apparaît que la fonctionnalité particulière présente dans l'électrolyte (plus précisément, la fonction vinyloxy présente dans le cation de formule (I)) est apte à polymériser à la surface d'une électrode comportant du graphite et ainsi générer une couche protectrice, de manière à limiter voire supprimer le phénomène d'exfoliation inhérent à la mise en contact d'un liquide ionique avec du graphite.

En outre, grâce à cette fonction vinyloxy qui comporte des doublets libres au niveau de l'atome d'oxygène, il ressort que la couche protectrice formée à la surface de l'électrode présente également une bonne aptitude pour véhiculer des ions lithium dans son épaisseur. Ce phénomène se traduit par une capacité réelle du système de stockage électrochimique plus proche de la capacité nominale d'une électrode comportant du graphite, en comparaison à un électrolyte comportant, comme liquide ionique, uniquement un liquide ionique conventionnel.

Ainsi, l'invention a trait également à un dispositif de stockage électrochimique (par exemple, une batterie ou un accumulateur) comprenant au moins une cellule comprenant une électrode positive et une électrode négative séparées l'une de l'autre par un séparateur comprenant, un électrolyte conforme à l'invention. Plus spécifiquement, l'une au moins desdites électrodes est une électrode comprenant, comme matériau actif, du graphite.

Avant d'entrer plus en détail dans la description des dispositifs de stockage électrochimique, nous précisons les définitions suivantes.

Par électrode positive, on entend, classiquement, dans ce qui précède et ce qui suit, l'électrode qui fait office de cathode, quand l'accumulateur débite du courant (c'est-à-dire lorsqu'il est en processus de décharge) et qui fait office d'anode lorsque l'accumulateur est en processus de charge.

Par électrode négative, on entend, classiquement, dans ce qui précède et ce qui suit, l'électrode qui fait office d'anode, quand l'accumulateur débite du courant (c'est-à-dire lorsqu'il est en processus de décharge) et qui fait office de cathode, lorsque l'accumulateur est en processus de charge.

On précise que chacune des électrodes comporte un matériau actif, c'est-à-dire un matériau qui est directement impliqué dans les réactions d'insertion et de désinsertion du lithium au cours des réactions de charge ou de décharge.

Outre la présence d'un matériau actif, l'électrode peut comprendre un liant polymérique, tel que du polyfluorure de vinylidène (connu sous l'abréviation PVDF), un mélange carboxyméthylcellulose (connu sous l'abréviation CMC) avec un latex du type styrène-butadiène (connu sous l'abréviation SBR) ou avec de l'acide polyacrylique (connu sous l'abréviation PAA) ainsi que un ou plusieurs adjuvants conducteurs de l'électricité, qui peuvent être des matériaux carbonés comme du noir de carbone.

Aussi, d'un point de vue structural, l'électrode peut se présenter sous forme d'un matériau composite comprenant une matrice en liant(s) polymérique(s), tel(s) que du PVDF (par exemple, à hauteur de 1 à 10 % de l'encre déposée sur le collecteur et précurseur du matériau composite), au sein de laquelle sont dispersées des charges constituées par le matériau actif (par exemple, à hauteur de 80 à 95% de masse de l'encre déposée sur le collecteur et précurseur du matériau composite) et éventuellement le ou les adjuvants conducteurs de l'électricité, tel que du noir de carbone (par exemple, à hauteur de 1 à 8% de la masse de l'encre déposée sur le collecteur et précurseur du matériau composite), ledit matériau composite étant déposé sur un collecteur de courant.

Le collecteur de courant peut être un collecteur en cuivre, lorsque l'électrode est une électrode négative, tandis que le collecteur de courant peut être un collecteur en aluminium, lorsque l'électrode est une électrode positive.

Plus spécifiquement, l'électrode comprenant du graphite peut être l'électrode négative, auquel cas le dispositif de stockage électrochimique peut être avantageusement un accumulateur du type lithium-ion.

Dans ce cas, l'électrode positive peut comprendre, comme matériau actif, du LiMnO₂, du LiMn₂O₄, du LiCoO₂, du LiNiO₂, du LiNi₁₋ₓMn₁₊ₓO₄ avec 0<x<1, du LiNi₁₋ₓCoₓO₂ avec 0<x<1, du LiNiₓMn_{y}Co_{z}O₂ avec 0<x,y,z<1 et x+y+z=1 ou du LiFePO₄.

Selon une autre variante, l'électrode comprenant du graphite peut être l'électrode positive.

Dans ce cas, l'électrode négative peut être une feuille métallique, telle qu'une feuille de lithium, ce qui signifie, dans ce cas, que l'électrode ne se présentera pas sous forme d'un matériau composite.

En alternative, l'électrode négative peut comprendre, comme matériau actif, du LiTiO₂ ou du Li₄Ti₅O₁₂, cette électrode négative pouvant se présenter, dans ce cas, sous forme d'un matériau composite, comme cela est défini plus haut.

Le séparateur peut être en un matériau poreux, tel qu'un matériau polymérique ou un matériau en fibres de verre, apte à accueillir dans sa porosité les électrolytes de l'invention. Il est entendu que le séparateur doit être mouillable et insoluble dans les électrolytes de l'invention. Un exemple de séparateur peut être du Celgard 2400®.

Plus spécifiquement, un dispositif conforme à l'invention est un dispositif, dans lequel l'électrode positive comprend, comme matériau actif, du graphite et dans lequel l'électrode négative est une électrode en lithium métallique.

L'électrolyte peut être quant à lui un électrolyte comprenant :
- un liquide ionique de formule (VI) telle que définie ci-dessus ;
- un liquide ionique de formule (X) telle que définie ci-dessus ;
- un sel de lithium *bis*(trifluorométhanesulfonyl)imidure de lithium.

Avec ce type de dispositif, les performances suivantes peuvent être atteintes :
- Capacité de première décharge supérieure à 25% de la capacité théorique de l'électrode négative ;
- Perte de capacité sur 10 cycles inférieure à 50% de la capacité du premier cycle ;
- Efficacité coulombique (énergie restituée à la décharge) d'au moins 90% au dixième cycle.

D'autres caractéristiques et avantages de l'invention apparaîtront du complément de description qui suit et qui se rapporte à des modes de réalisation particuliers.

Bien entendu, ce complément de description n'est donné qu'à titre d'illustration de l'invention et n'en constitue en aucun cas une limitation.

### EXPOSÉ DÉTAILLÉ DE MODES DE RÉALISATION PARTICULIERS

### EXEMPLE 1

Cet exemple illustre la préparation d'un électrolyte conforme à l'invention.

Cet électrolyte est réalisé en boîte à gants par mise en commun de 0,9 mL de *bis*(trifluorométhanesulfonyl)imidure de N,N-méthylpropylpyrrolidinium de formule (X) suivante : 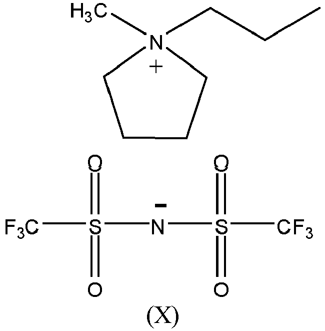 avec 0,1 mL de *bis*(fluorosulfonyl)imidure de N,N-méthyl-(2-vinyloxyéthyl)pyrrolidinium de formule (VI) suivante : 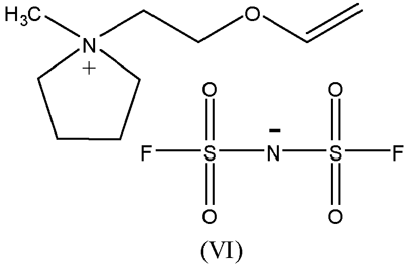 et 0,2871 g de *bis*(trifluorométhanesulfonyl)imidure de lithium (LiTFSI).

### EXEMPLE COMPARATIF 1

Cet exemple illustre la préparation d'un électrolyte non conforme à l'invention.

Cet électrolyte est réalisé en boîte à gants par mise en commun de 1,0 mL de *bis*(trifluorométhanesulfonyl)imidure de N,N-méthylpropylpyrrolidinium avec 0,2871 g de *bis*(trifluorométhanesulfonyl)imidure de lithium (LiTFSI).

### EXEMPLE 2

Dans cet exemple, les performances des électrolytes mentionnés à l'exemple 1 et l'exemple comparatif 1 sont évaluées pour une application en stockage électrochimique. Pour ce faire, des cellules de tests (plus spécifiquement, des piles boutons de format 2032) sont préalablement assemblées en boîte à gants selon le protocole suivant :
- Mise en place d'un carter de pile 2032 (capot inférieur) muni d'un joint d'étanchéité ;
- Insertion d'un disque en inox d'un diamètre ajusté au diamètre intérieur de la pile ;
- Insertion d'un disque de diamètre de 16 mm d'une électrode positive comprenant, comme matériau actif, du graphite (96 %), du noir de carbone Super P (1 %), un liant comprenant du carboxyméthylcellulose (1 %) et un caoutchouc styrène/butadiène (2 %), d'une capacité de 1,4 mAh/cm², dont la face comprenant du matériau est tournée vers le haut ;
- Insertion d'un séparateur Whatman de 16,5 mm de diamètre préalablement imbibé de 150 µL de l'un des électrolytes susmentionnés ;
- Insertion d'un disque de lithium de diamètre 16 mm, qui fait office d'électrode négative ;
- Insertion d'un second disque en inox et d'un ressort de compression ;
- Ajout d'un capot supérieur puis sertissage par pressage de l'ensemble.

Une fois réalisées, les piles boutons sont introduites dans un banc de cyclage de type Biologie™ positionné dans une étuve. Les conditions de cyclage suivantes sont ensuite appliquées :
- Température de test consignée à 45 °C ;
- Réalisation de 10 cycles successifs de charge/décharge par chronopotentiométrie selon les paramètres suivants :
   ∘ Charge de 1,5 V à 0,02 V en 10 heures (taux de charge C/10) ;
   ∘ Décharge de 0,02 V à 1,5 V en 10 heures (taux de décharge D/10) ;
- Réalisation de 10 cycles successifs de charge/décharge par chronopotentiométrie selon les paramètres suivants :
   ∘ Charge de 1,5 V à 0,02 V en 5 heures (taux de charge C/5) ;
   ∘ Décharge de 0,02 V à 1,5 V en 5 heures (taux de décharge D/5).

L'évolution des capacités de piles mesurées lors de ce protocole, ainsi que l'efficacité de restitution de l'énergie stockée (efficacité coulombique), sont retranscrites dans les tableaux suivants pour les différents électrolytes testés.
*Cyclages à 45 °C, à un taux de décharge de D/10 (décharge complète en 10 heures)

| Electrolyte | % de la capacité de l'électrode graphite* (cycle 1 / cycle 10) | Perte de capacité** (%) | Efficacité coulombique*** (%) |
|---|---|---|---|
| Exemple comparatif 1 | 3,2/0,9 | 71,7 | 97 |
| Exemple 1 | 71,4/71,4 | 0 | 100 |

| | | | |
|---|---|---|---|
| * capacité de l'électrode en graphite = 1,4 mAh/cm² ** mesurée entre les cycles 1 et 10 *** au cycle 10 | | | |

*Cyclages à 45 °C, à un taux de décharge de D/5 (décharge complète en 5 heures)

| Electrolyte | % de la capacité de l'électrode graphite* (cycle 1 / cycle 10) | Perte de capacité** (%) | Efficacité coulombique*** (%) |
|---|---|---|---|
| Exemple comparatif 1 | 0,7/0,6 | 15,2 | 99 |
| Exemple 1 | 70,3 / 69,3 | 1,5 | 100 |

| | | | |
|---|---|---|---|
| * capacité de l'électrode en graphite = 1,4 mAh/cm² ** mesurée entre les cycles 1 et 10 *** au cycle 10 | | | |

Au regard de ces résultats, de par la capacité de cellule la plus proche possible de la capacité de l'électrode en graphite (jusqu'à 71,4 %), les faibles pertes de capacité mesurées (jusqu'à 0% sur 10 cycles) et l'efficacité coulombique supérieure à 90%, l'électrolyte de l'invention fait état de performances en rupture par rapport aux électrolytes à base de liquides ioniques conventionnels, ce qui confirme l'intérêt présenté par les électrolytes de l'invention.

## Revendications

1. Electrolyte comprenant au moins un sel de lithium et au moins deux liquides ioniques, dont au moins l'un est un liquide ionique résultant de l'association d'au moins un cation répondant à la formule (I) suivante : 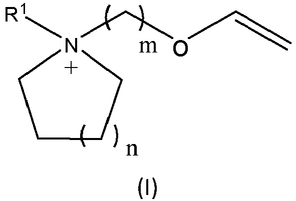 dans laquelle :
- R¹ est un groupe hydrocarboné acyclique ;
- n est un entier allant de 0 à 3 ;
- m est un entier allant de 1 à 4 ;
et d'au moins ; un anion Y.

2. Electrolyte selon la revendication 1, dans lequel le cation de formule (I) répond à la formule spécifique (Ib) suivante : 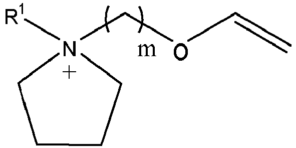 dans laquelle R¹ et m sont tels que définis à la revendication 1.

3. Electrolyte selon la revendication 1 ou 2, dans lequel R¹ est un groupe alkyle comprenant de 1 à 4 atomes de carbone.

4. Electrolyte selon l'une quelconque des revendications précédentes, dans lequel m est égal à 2.

5. Electrolyte selon l'une quelconque des revendications précédentes, dans lequel Y est un anion choisi parmi un anion nitrate, un anion phosphate, les anions imidures.

6. Electrolyte selon l'une quelconque des revendications précédentes, dans lequel Y est un anion choisi parmi les anions imidures.

7. Electrolyte selon l'une quelconque des revendications précédentes, dans lequel l'anion Y est un anion imidure répondant à la formule (II') suivante : 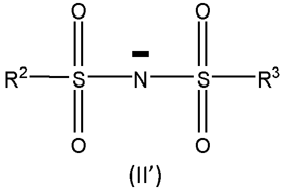 dans laquelle R² et R³ représentent, indépendamment l'un de l'autre, un atome de fluor, un groupe perfluorocarboné.

8. Electrolyte selon l'une quelconque des revendications précédentes, dans lequel l'un au moins des liquides ioniques est un liquide ionique résultant de l'association d'un cation phosphonium, sulfonium, azétidinium, pyrrolidinium ou pipéridinium et d'un anion halogénure, phosphate, nitrate ou imidure, étant entendu que, lorsque le cation est un cation azétidinium, pyrrolidinium ou pipéridinium, le cation ne répond pas à la formule (I) définie à la revendication 1.

9. Electrolyte selon la revendication 8, dans lequel le cation est un cation pyrrolidinium ou pipéridinium.

10. Electrolyte selon la revendication 8 ou 9, dans lequel le cation répond à la formule (VIII) suivante : 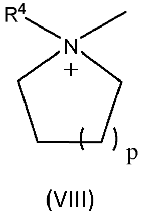 dans laquelle :
- R⁴ est un groupe hydrocarboné acyclique ; et
- p est un entier allant de 0 à 2.

11. Electrolyte selon l'une quelconque des revendications 8 à 10, dans lequel le cation répond à la formule (VIIIb) : 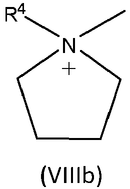 dans laquelle R⁴ est tel que défini à la revendication 10.

12. Electrolyte selon l'une quelconque des revendications 8 à 11, dans lequel l'anion est un anion imidure.

13. Electrolyte selon l'une quelconque des revendications 8 à 12, dans lequel le liquide ionique répond à la formule (X) suivante :

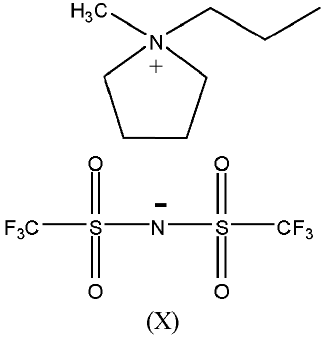

14. Dispositif de stockage électrochimique comprenant au moins une cellule comprenant une électrode positive et une électrode négative séparées l'une de l'autre par un séparateur comprenant, un électrolyte tel que défini selon l'une quelconque des revendications 1 à 13.

15. Dispositif de stockage électrochimique selon la revendication 14, dans lequel l'une au moins des électrodes est une électrode comprenant, comme matériau actif, du graphite.

## Patentansprüche

1. Elektrolyt, enthaltend zumindest ein Lithiumsalz und zumindest zwei ionische Flüssigkeiten, von denen zumindest eine eine ionische Flüssigkeit ist, die aus der Assoziation von zumindest einem Kation der folgenden Formel (I): 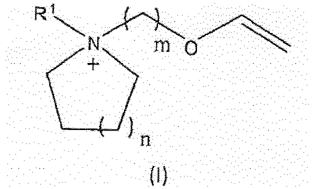 worin:
- R¹ eine azyklische Kohlenwasserstoffgruppe ist;
- n eine ganze Zahl zwischen 0 und 3 ist;
- m eine ganze Zahl zwischen 1 und 4 ist;
und zumindest einem Anion Y resultiert.

2. Elektrolyt nach Anspruch 1, wobei das Kation der Formel (I) der folgenden spezifischen Formel (Ib) entspricht: 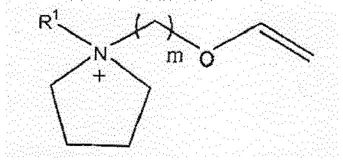 worin R¹ und m wie in Anspruch 1 definiert sind.

3. Elektrolyt nach Anspruch 1 oder 2, wobei R¹ eine Alkylgruppe mit 1 bis 4 Kohlenstoffatomen ist.

4. Elektrolyt nach einem der vorangehenden Ansprüche, wobei m gleich 2 ist.

5. Elektrolyt nach einem der vorangehenden Ansprüche, wobei Y ein Anion ist, ausgewählt aus einem Nitratanion, einem Phosphatanion, Imidanionen.

6. Elektrolyt nach einem der vorangehenden Ansprüche, wobei Y ein Anion ist, das aus Imidanionen ausgewählt ist.

7. Elektrolyt nach einem der vorangehenden Ansprüche, wobei das Anion Y ein Imidanion der folgenden Formel (II') ist: 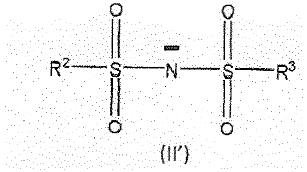 worin R² und R³ unabhängig voneinander ein Fluoratom, eine Perfluorkohlenstoffgruppe darstellen.

8. Elektrolyt nach einem der vorangehenden Ansprüche, wobei zumindest eine der ionischen Flüssigkeiten eine ionische Flüssigkeit ist, die aus der Assoziation eines Phosphonium-, Sulfonium-, Azetidinium-, Pyrrolidinium- oder Piperidinium-Kations und eines Halogenid-, Phosphat-, Nitrat- oder Imid-Anions resultiert, mit der Maßgabe, dass, wenn das Kation ein Azetidinium-, Pyrrolidinium- oder Piperidinium-Kation ist, das Kation nicht der in Anspruch 1 definierten Formel (I) entspricht.

9. Elektrolyt nach Anspruch 8, wobei das Kation ein Pyrrolidinium- oder Piperidinium-Kation ist.

10. Elektrolyt nach Anspruch 8 oder 9, wobei das Kation der folgenden Formel (VIII) entspricht: 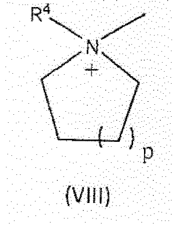 worin:
- R⁴ eine azyklische Kohlenwasserstoffgruppe ist; und
- p eine ganze Zahl von 0 bis 2 ist.

11. Elektrolyt nach einem der Ansprüche 8 bis 10, wobei das Kation der Formel (Vlllb) entspricht: 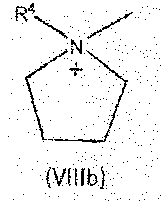 worin R⁴ wie in Anspruch 10 definiert ist.

12. Elektrolyt nach einem der Ansprüche 8 bis 11, wobei das Anion ein ImidAnion ist.

13. Elektrolyt nach einem der Ansprüche 8 bis 12, wobei die ionische Flüssigkeit der folgenden Formel (X) entspricht:

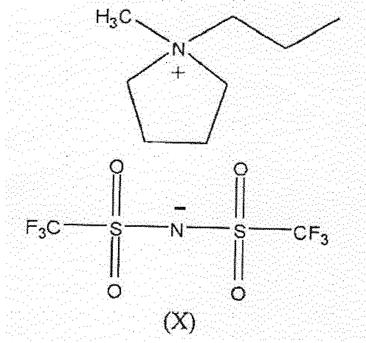

14. Elektrochemische Speichervorrichtung, enthaltend zumindest eine Zelle, die eine positive Elektrode und eine negative Elektrode enthält, die durch einen Separator voneinander getrennt sind, der einen Elektrolyten nach einem der Ansprüche 1 bis 13 enthält.

15. Elektrochemische Speichervorrichtung nach Anspruch 14, wobei zumindest eine der Elektroden eine Elektrode ist, die als aktives Material Graphit enthält.

## Claims

1. Electrolyte comprising at least one lithium salt and at least two ionic liquids, at least one of which is an ionic liquid resulting from the association of at least one cation complying with the following formula (I): 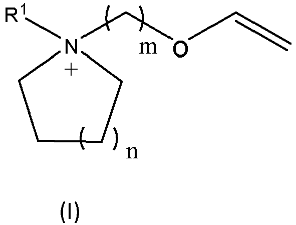 In which:
- R¹ is an acyclic hydrocarbon group;
- n is an integer ranging from 0 to 3;
- m is an integer ranging from 1 to 4;
and at least one Y anion.

2. Electrolyte according to claim 1, wherein the cation of formula (I) complies with the following specific formula (Ib): 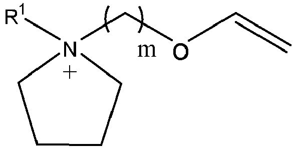 in which R¹ and m are as defined in claim 1.

3. Electrolyte according to claim 1 or claim 2, wherein R¹ is an alkyl group comprising 1 to 4 carbon atoms.

4. Electrolyte according to any of the preceding claims, wherein m is equal to 2.

5. Electrolyte according to any of the preceding claims, wherein Y is an anion chosen from a nitrate anion, a phosphate anion or imidide anions.

6. Electrolyte according to any of the preceding claims, wherein Y is an anion chosen from imidide anions.

7. Electrolyte according to any of the preceding claims, wherein the Y anion is an imidide anion complying with the following formula (II'): 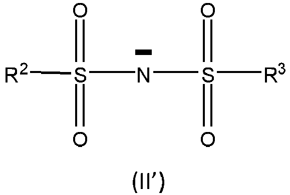 wherein R² and R³ represent, independently of one another, a fluorine atom, a perfluorocarbon group.

8. Electrolyte according to any of the preceding claims, wherein the at least one of the ionic liquids is an ionic liquid resulting from the association of a phosphonium, sulfonium, azetidinium, pyrrolidinium or piperidinium cation and a halide, phosphate, nitrate or imidide anion, it being understood that, when the cation is an azetidinium, pyrrolidinium or piperidinium cation, the cation does not comply with the formula (I) defined in claim 1.

9. Electrolyte according to claim 8, wherein the cation is a pyrrolidinium or piperidinium cation.

10. Electrolyte according to claim 8 or 9, wherein the cation complies with the following formula (VIII): 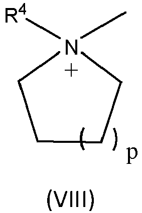 in which:
- R⁴ is an acyclic hydrocarbon group; and
- p is an integer ranging from 0 to 2.

11. Electrolyte according to any of claims 8 to 10, wherein the cation complies with formula (Vlllb): 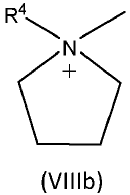 wherein R⁴ is as defined in claim 10.

12. Electrolyte according to any of claims 8 to 11, wherein the anion is an imidide anion.

13. Electrolyte according to any of claims 8 to 12, wherein the ionic liquid complies with the following formula (X):

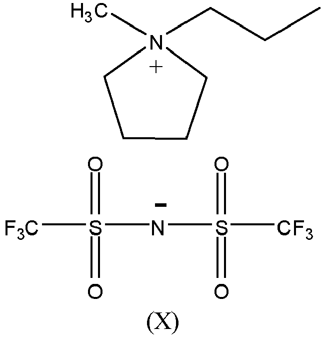

14. Electrochemical storage device comprising at least one cell comprising a positive electrode and a negative electrode separated from one another by a separator comprising an electrolyte as defined according to any of claims 1 to 13.

15. Electrochemical storage device according to claim 14, wherein the at least one of the electrodes is an electrode comprising graphite as an active material.
